# EUROPEAN PATENT SPECIFICATION

(11) **EP 0 584 956 B1**
(45) Date of publication and mention of the grant of the patent: **17.01.1996**
(21) Application number: 93305877.8
(22) Date of filing: 26.07.1993
(51) Int. Cl.: C07D 301/19

(54) **Preparation of mono epoxides and tertiary butyl alcohol using regenerated catalyst**
Verfahren zur Herstellung von Monoepoxiden und tert. Butylalkohol mit einem regeneriertem Katalysator
Préparation de mono-époxides et d'alcoole tert. butylique utilisant un catalyseur régénéré

(30) Priority: 24.08.1992 US 933836
(43) Date of publication of application: 02.03.1994
(73) Proprietor: TEXACO CHEMICAL INC., Houston, Texas 77056 (US)
(72) Inventor: Marquis, Edward Thomas, Austin TX 78758 (US); Meyer Robert Allen, Ballwin MO 63021 (US)
(74) Representative: Green, Mark Charles

(56) References cited:
- US-A- 3 931 044
- US-A- 4 661 463
- US-A- 5 101 052
- US-A- 5 107 067

## Description

This invention relates to an improvement in the method for preparing an epoxide of an alpha olefin such as propylene oxide and tertiary butyl alcohol from a C₃ to C₁₂ alpha olefin such as propylene and tertiary butyl hydroperoxide. More particularly, this invention relates to a method for the preparation of propylene oxide and tertiary butyl alcohol from propylene and tertiary butyl hydroperoxide using a regenerated catalyst. Also, this invention relates to a process for preparing an epoxide of an alpha olefin such as propylene oxide and tertiary butyl alcohol by the molybdenum catalyzed reaction of a C₃ to C₁₂ linear alpha olefin such as octene-1 or propylene with tertiary butyl hydroperoxide wherein a distillation fraction obtained from the epoxidation reaction mixture which contains residual molybdenum catalyst is reacted with ammonia in liquid phase to provide a precipitate which is then reacted with ethylene glycol to provide a regenerated molybdenum catalyst which is used as a portion of the catalyst requirement to catalyze the reaction of the linear alpha olefin with tertiary butyl hydroperoxide.

Kollar U. S. Patent No. 3,350,422 and U. S. Patent No. 3,351,635 disclose and describe a method for the preparation of olefin epoxides and alcohols by the molybdenum catalyzed reaction of an olefin with a tertiary alkyl peroxide. It was recognized early on, as exemplified by Kollar U. S. Patent No. 3,860,862, Kollar U. S. Patent No. 3,947,500 and Kollar U. S. Patent No. 3,947,501 that side reactions occur during the epoxidation of an olefin with a hydroperoxide in the presence of a soluble molybdenum catalyst that give rise to oxygen-containing by-products including acids, esters and ketones. In Kollar U. S. Patents No. 3,860,662, No. 3,947,500 and No. 3,947,501, Kollar sought to improve the efficiency of the epoxidation reaction by reducing the acidic characteristics of the epoxidation reaction product by adding a basic material to the reaction mixture, by adding a chemical reducing agent to the reaction mixture or by hydrogenating the reaction mixture in the presence of a hydrogenation catalyst.

In British Patent No. 1,298,253, it was proposed to conduct the reaction on a continuous basis using an excess of tertiary butyl hydroperoxide in a first reaction zone and added propylene in a second reaction zone.

More recently, Marquis et al. in U. S. Patent No. 4,891,437 have proposed to improve the efficiency of the process and to reduce by-product formation by the molybdenum catalyst catalyzed reaction of an olefin with tertiary hydroperoxide or tertiary butyl hydroperoxide in a reaction medium containing more than about 60 wt.% of polar components. Marquis et al. in U. S. Patent No. 4,626,596 have also proposed the use of a molybdenum/ alkylene glycol complex prepared in a described manner in the preparation of propylene oxide and tertiary butyl alcohol from propylene and tertiary butyl hydroperoxide. Marquis et al. have also proposed the use of a molybdenum/sodium/ethylene glycol catalyst for the reaction in U. S. Patent No. 4,845,251.

It was also recognized early on that the manner in which the molybdenum catalyst was prepared would have an important bearing on the efficiency of the reaction. Thus, in U. S. Patent No. 3,362,972, Kollar proposed the preparation of molybdenum catalysts by the reaction of a molybdenum salt with a carboxylic acid.

Subsequently, Marquis et al. sought to provide improved molybdenum catalysts such as catalysts prepared in accordance with U. S. Patent No. 4,626,596 by the reaction of an ammonium-containing molybdenum compound with an alkylene glycol in the presence of a controlled amount of water under recited reaction conditions. Marquis et al. also disclose the preparation of storage stable molybdenum/alkanol complexes by reacting an ammonium molybdate with an alkanol in the presence of controlled amounts of water under recited reaction conditions in U. S. Patent No. 4,650,886. Marquis et al. in U. S. Patent No. 4,654,427 further describe the preparation of storage stable solutions of molybdenum/alkanol complexes by reacting a molybdenum oxide, ammonium hydroxide, and a straight chain or branched chain C₆-C₁₃ alkanol. In U. S. Patent No. 4,703,027, Marquis et al. disclose complexes prepared by reacting a solid ammonium molybdate and a solid alkali metal molybdate with ethylene glycol under controlled reaction conditions. Also, in Marquis et al. U. S. Patent No. 4,758,681, a method of preparing a molybdenum catalyst is disclosed wherein ethylene glycol is reacted with an ammonium dimolybdate under recited reaction conditions. Also, in U. S. Patent No. 5,107,068, Marquis et al. propose the preparation of a catalyst by the reaction of an ammonium-containing molybdenum compound with an alkylene glycol in the presence of water followed by mild stripping of water subsequent to the formation of the molybdenum/alkylene glycol complex.

It is conventional practice to charge the epoxidation reaction mixture to a distillation zone and there separate it into desired distillation fractions such as a recycle propylene fraction, a product propylene oxide fraction, a tertiary butyl alcohol fraction and a heavy distillation fraction containing oxygenated by-products such as acids, esters and ketones and catalyst residue. The presence of the molybdenum compounds in this heavy distillation fraction represents a problem insofar as further workup or disposal of the fraction is concerned.

It was known from the workup of molybdenum-catalyzed coal liquefaction reaction products that molybdenum could be recovered by treating the process residue with an alkali to form water soluble molybdates which could then be roasted and leached to form a product which could thereafter be extracted with water, acidified and ammoniated to form a molybdenum-bearing precipitate which could be dissolved in ammonium hydroxide to form a solution to be used to catalyze coal liquefaction. See, for example, Sebenik et al. U. S. Patent No. 4,374,100.

Meyer et al. in U. S. Patent No. 5,093,509 propose to recover molybdenum by a process that uses a synthetic high surface area amorphous magnesium silicate as a solid adsorbent. In U. S. Patent No. 5,101,052, Meyer et al. propose to recover molybdenum by precipitating molybdenum from a residual heavy distillation fraction with ammonia. Smith et al. in U. S. Patent No. 5,128,492 describe molybdenum recovery by hydrogenation. British Patent No. 1,317,480 also proposes to recover molybdenum compounds by a process involving the use of water or aqueous ammonia to treat the heavy distillation fraction in order to form an aqueous phase containing molybdenum from which the molybdenum can subsequently be recovered as molybdenum trioxide.

Marquis et al. in U. S. Patent No. 5,093,506 disclose a process wherein a distillation fraction containing tertiary butyl hydroperoxide, tertiary butyl alcohol, and carboxylic acid contaminants is partially neutralized with calcium oxide or calcium hydroxide to form a precipitate which is removed and the resultant supernatant liquid is then recycled to the epoxidation reactor for use as an oxidant/solvent without causing precipitation of molybdenum in the reaction zone.

Mocella in US-A-4661463 describes a process of regenerating a soluble, stable molybdenum-containing catalyst suitable for epoxidation of olefins with a hydroperoxide which comprises thermally precipitating and separating a molybdenum-containing solid obtained from a spent catalyst stream derived from a molybdenum catalyzed epoxidation of an olefin and solubilizing the precipitated solid by contacting with a liquid composition comprising an admixture of a monohydroxy alcohol, a polyhydroxy alcohol and an organic peroxide or hydroperoxide, said polyhydroxy alcohol being present in an amount of at least about 2 moles per mole or molybdenum to be solubilized.

Maurin in US-A-3931044 describes a method regenerating catalysts used in diol synthesis. Molybdenum catalyst residue is treated with ammonia and the diol acts on the ammonium molybdate so obtained. Maurin does not disclose the treating sequence whereby a substantially solids-free ethylene glycol solution of a complex of ethylene glycol with the ammonium-containing compounds in the solid ammonium-containing molybdenum precipitate is obtained.

The present invention provides a process according to Claim 1.

More particularly, the process of the present invention is a process wherein:
a. anhydrous ethylene glycol and anhydrous ammonium dimolybdate are charged to a catalyst preparation reactor in an amount sufficient to provide about 7 to about 20 moles of ethylene glycol per gram atom of molybdenum contained in the ammonium dimolybdate;
b. the resultant mixture is heated at a pressure of 0.1 to 20.8 MPa (0 to 3,000 psig) in a reaction temperature of 70° to 250°C. for 1 to 2 hours in order to form a solids-free solution of a complex of ethylene glycol with ammonium dimolybdate;
c. the solids-free solution of the complex of ethylene glycol with the ammonium dimolybdate is held at a temperature of 90° to 110°C. for a time within the range of 0.5 to 5 hours sufficient to permit vaporization and removal of volatile by-products, including water, by an amount sufficient to provide an initial ethylene glycol solution containing 16 to 52 wt.% of said complex of ethylene glycol with said ammonium dimolybdate and having a molybdenum content of 6 to 20 wt.% and a water concentration of 0.5 to 6 wt.%;
d. a tertiary butyl alcohol solution of tertiary butyl hydroperoxide and propylene charged to an epoxidation reaction zone together with a catalytic amount of a catalyst mixture composed of said initial ethylene glycol solution of said complex with ethylene glycol with said ammonium dimolybdate and a final ethylene glycol solution of a complex of ethylene glycol with ammonium-containing molybdenum compounds derived from a precipitate of solid ammonium containing molybdenum compounds, the initial molar ratio of propylene to tertiary butyl alcohol solution of active tertiary butyl hydroperoxide being 1.05:1 to 2:1 and containing 100 to 600 ppm of catalyst mixture;
e. establishing epoxidation reaction conditions in the epoxidation reaction zone including a temperature of 500 to 180°C., and a residence time of 1 to 5 hours to thereby react the propylene with the tertiary butyl hydroperoxide to provide an epoxidation reaction product comprising unreacted propylene, unreacted tertiary butyl hydroperoxide, propylene oxide, tertiary butyl alcohol, dissolved molybdenum ethylene glycol catalyst complex and oxygen-containing impurities;
f. resolving the epoxidation reaction products in a distillation zone into distillation fractions including a distillate propylene fraction, a distillate propylene oxide fraction, a distillate tertiary butyl alcohol fraction and a heavy liquid distillation fraction composed primarily of tertiary butyl hydroperoxide, tertiary butyl alcohol, oxygen-containing impurities and dissolved molybdenum-ethylene glycol catalyst complex;
g. charging the heavy distillation fraction to a precipitation zone and saturating it therein with ammonia to form a liquid amination product containing a precipitate of solid ammonium-containing molybdenum compounds;
h. charging the amination product to a separation zone and therein separating the solid ammonium-containing molybdenum precipitate from the liquid medium;
i. recovering the solid ammonium-containing molybdenum precipitate;
j. charging the recovered solid ammonium-containing molybdenum precipitate to a catalyst regeneration zone and mixing it therein with an amount of substantially anhydrous ethylene glycol sufficient to provide 7 to 20 moles of ethylene glycol per gram atom of molybdenum contained in the solid ammonium-containing molybdenum precipitate to form an ethylene glycol feed mixture;
k. heating the ethylene glycol feed mixture in a catalyst regeneration zone at a pressure of 0.1 to 20.07 MPa (0 to 3,000 psig) and a reaction temperature of 70° to 250°C. for a time within the range of 1 to 2 hours sufficient to form a substantially solids-free ethylene glycol solution of a complex of ethylene glycol with ammonium-containing compounds in said solid ammonium-containing molybdenum precipitate;
1. holding said solids-free solution of said complex of ethylene glycol with said ammonium-containing compounds in said catalyst regeneration zone at a temperature of 90° to 110°C. for a time, within the range of 0.5 to 5 hours, sufficient to permit vaporization and removal of volatile by-products, including water, by an amount sufficient to provide a final ethylene glycol solution containing 16 to 52 wt.% of said complex of ethylene glycol with said ammonium-containing molybdenum compounds and having a molybdenum content of 6 to 20 wt.% and a water concentration of 0.5 to 6%; and
m. charging said thus-prepared final ethylene glycol solution to said epoxidation reaction zone as said final ethylene glycol solution of a complex of ethylene glycol with ammonium-containing molybdenum compounds derived from a precipitate of solid ammonium-containing molybdenum compounds.

Still more preferably, the process of the present invention is conducted in the above described manner on a continuous basis.

### Starting Materials

The starting materials for the present invention are a C₃-C₁₂ linear alpha mono olefin, tertiary butyl hydroperoxide, ethylene glycol, an initial ethylene glycol solution of a complex of ethylene glycol with ammonium dimolybdate and a final ethylene glycol solution of a complex of ethylene glycol with ammonium-containing molybdenum compounds derived from a precipitate of solid ammonium-containing molybdenum compounds.

The linear alpha olefins to be used as feedstocks in accordance with the present invention may be unbranched linear mono olefins containing from 3 to 12 carbon atoms in the molecule including compounds such as propylene, butene-1, pentene-1, hexene-1, octene-1, dodecene-1, etc. A preferred mono olefin is propylene.

The tertiary butyl hydroperoxide that is used may be a standard industrial grade of tertiary butyl hydroperoxide prepared, for example, by the oxidation of isobutane with oxygen to provide as a reaction product, a solution of tertiary butyl hydroperoxide and tertiary butyl alcohol.

The ethylene glycol is preferably an industrial grade of ethylene glycol that is substantially anhydrous. An industrial grade of ammonium dimolybdate may also be used.

### Preparation of the Initial Ethylene Glycol Solution

The starting materials for the preparation of the initial ethylene glycol solution are ethylene glycol and ammonium dimolybdate. The substantially anhydrous ethylene glycol and ammonium dimolybdate are charged to a catalyst preparation zone in amounts sufficient to provide a molar ratio of about 7 to about 20 moles of ethylene glycol per gram atom of molybdenum contained in the ammonium dimolybdate. The resultant feed mixture is heated at a temperature of about 80° to about 130°C. and more preferably at a temperature of about 90° to about 100°C. at a pressure of 0.1 to 20.7 MPa (about 0 to about 3,000 psig) and preferably about 0.1 MPa (0 psig) to thereby form a solution of a complex of ethylene glycol with the ammonium dimolybdate in ethylene glycol. The resultant solution is held at a temperature of about 90° to about 120°C. for about 0.5 to about 5 hours sufficient to permit volatilization of volatile reaction components including water and ammonia so as to provide an initial ethylene glycol solution containing about 16 to about 52 wt.% of the complex of ethylene glycol with the ammonium dimolybdate and having a molybdenum content of about 6 to about 20 wt.% and a water concentrate of about 0.5 to about 6 wt.%.

### Preparation of the Final Ethylene Glycol Complex

The final ethylene glycol complex is also prepared in the manner described above for the preparation of the initial ethylene glycol solution. However, the source of the molybdenum is not the ammonium dimolybdate specified above but, instead, is a precipitate of a solid ammonium-containing molybdenum compound prepared and obtained in a manner to be described.

### Epoxidation

In general, the alpha olefin is reacted with tertiary butyl hydroperoxide in solution in tertiary butyl alcohol in the presence of a catalyst mixture composed of the initial ethylene glycol solution and the final ethylene glycol solution by a process of the type disclosed in Kollar U. S. Patent No. 3,351,635, such as a process of the type disclosed in Marquis et al. U. S. Patents No. 4,845,251, No. 4,891,437 or No. 5,107,067.

In particular, the alpha olefin will be used in the form of an anhydrous olefin and the tertiary butyl hydroperoxide will be charged in the form of a solution of about 40 to about 75 wt.% of tertiary butyl hydroperoxide in tertiary butyl alcohol. The reactants will be charged in amounts such that the reaction mixture contains from about 200 to about 600 ppm of the solubilized molybdenum catalyst and such that the molar ratio of alpha olefin to tertiary butyl hydroperoxide is within the range of about 1.05:1 to about 2:1 and, more preferably, within the range of from about 1.05:1 to about 1.8:1, and still more preferably in the range of from about 1.05:1 to about 1.35:1. When the feed materials to the epoxidation reaction zone are charged in the described fashion, the resultant reaction mixture will initially contain more than about 60 wt.% of polar components (tertiary butyl alcohol and tertiary butyl hydroperoxide). The olefin epoxide that is formed during the epoxidation reaction is also a polar material.

The epoxidation reaction may suitably be conducted at a temperature within the range of about 50° to about 180°C., preferably within the range of about 90° to about 140°C., and more preferably within the range from about 100° to about 120°C.

The reaction is suitably conducted at a pressure sufficient to maintain the reactants in liquid phase. Thus, for the higher alpha olefins such as octene-1, the reaction can be conducted at atmospheric pressure. However, a superatmospheric pressure is required for the more volatile lower alpha olefins, such as propylene and butene-1. In this situation, the lower pressure is suitably 3.5 MPa (about 500 psig).

Higher pressures such as pressures within the range of 3.5 MPa to 20.7MPa (about 500 to about 3,000 psig) may be used if desired. Reaction time may suitably vary from about 0.5 to about 5 hours, and more preferably from about 1.5 to about 2 hours. The reaction may be conducted in a single reaction zone or in a plurality of reaction zones as described, for example, in Marquis et al. U. S. Patent No. 4,891,437.

### Molybdenum Recovery

In accordance with the present invention, the epoxidation reaction product formed in the above described fashion, is charged to a distillation zone and resolved therein into a plurality of fractions including a distillate olefin fraction, a distillate olefin epoxide fraction, a distillate tertiary butyl alcohol fraction, a heavy liquid distillation fraction containing tertiary butyl alcohol, oxygen-containing impurities and dissolved molybdenum-ethylene glycol catalyst complex.

The heavy distillation fraction may then be treated, as for example, in the manner disclosed in Meyer et al. U. S. Patent No. 5,101,052.

In accordance with the present invention, a heavy distillation fraction comprising tertiary butyl hydroperoxide, tertiary butyl alcohol and impurities including about 0.4 to about 0.8 wt.% of dissolved molybdenum catalyst and lower aliphatic carboxylic acids resulting from the removal of olefin, olefin epoxide and tertiary butyl alcohol from an epoxidation reaction product is charged to a precipitation zone which may suitably comprise a reactor, such as an autoclave, provided with suitable agitating means (e.g., an impeller), temperature control means such as a jacket or coils through which a liquid heat exchange medium can be circulated, charge lines for the heavy distillation fraction and for the ammonia and a discharge line for withdrawal of the treated product. Within the precipitation zone the ammonia will react with the molybdenum compounds present in the heavy distillation fraction to form a reaction product comprising a molybdenum-containing precipitate that can be withdrawn from the precipitation zone. The precipitate can be removed in any desired manner in a precipitate removal zone (e.g., by filtration, centrifugation, etc.).

It has been discovered in accordance with the present invention that when the heavy distillation fraction contains only about 0.8 wt.% or less of molybdenum (e.g., 0.4 to 0.8 wt.%), the precipitation of the molybdenum compounds will be essentially complete in that that precipitate will contain substantially all of the molybdenum charged to the precipitation zone. It has been discovered that when heavy distillation fractions containing larger amounts of molybdenum are used, an undesirable higher percentage of the molybdenum will remain dissolved in the treated heavy fraction.

The heavy distillation fraction will normally contain less than about 1 wt.% of water and, therefore, ammonia should be used, as such, rather than in the form of an aqueous ammoniacal solution. The ammonia should preferably be used in an amount which is equivalent to the amount of molybdenum in the heavy distillation fraction and, preferably, an excess of ammonia will be used, such as about 1 to about 200 moles of ammonia per gram atom of molybdenum present in the heavy distillation fraction. Preferably, the heavy distillation fraction is saturated with ammonia.

The precipitation reaction can be conducted under ambient conditions of temperature and pressure, although somewhat higher temperatures and pressures may be used, if desired, such as temperatures within the range of about 20° to 250°C. and pressures within the range of 0.1 to 20.7MPa (about 0 to 3,000 psig). The contact time should be sufficient to insure that the precipitation reaction goes to completion (e.g., 0.2 to 2 hours).

After the precipitation reaction is completed, the mixture of precipitate and treated heavy distillation fraction is withdrawn from the precipitation zone for removal of the precipitate. The precipitate can be removed in any desired manner, e.g., filtration, centrifugation, evaporation, etc. Since the precipitate constitutes only a minor amount of the mixture of precipitate and treated heavy distillation fraction, filtration is preferred.

The filtrate obtained by the practice of the present invention will contain only a residual amount of molybdenum (e.g., from 10 to 100 ppm). For example, it can be charged to a boiler as a fuel, or further treated (e.g., by vacuum distillation for the recovery of at least a portion of the tertiary butyl alcohol and/or tertiary butyl hydroperoxide contained therein.

The precipitate will normally contain about 40 to about 58 wt.% of molybdenum.

### Catalyst Regeneration

In accordance with the present invention, the solid ammonium-containing molybdenum precipitate is charged to a catalyst regeneration zone where it is mixed with substantially anhydrous ethylene glycol in an amount sufficient to provide about 7 to about 20 moles of ethylene glycol per gram atom of molybdenum contained in the solid ammonium-containing molybdenum precipitate to thereby form an ethylene glycol feed mixture. A preferred ratio of ethylene glycol to gram atoms of molybdenum is within the range of about 8:1 to about 16:1. The water content of the ethylene glycol feed mixture should preferably be within the range of about 0.1 to about 2 wt.%. The resultant mixture of ethylene glycol with precipitate is heated in the catalyst regeneration zone at a temperature within the range of about 50° to about 150°C., and more preferably within the range of about 90° to about 120°C. and a pressure of 0.1 to 20.7MPa (about 0 to about 3,000 psig), and preferably 0.1MPa (about 0 psig), for a period of time within the range of about 0.2 to 2 hours, and preferably 0.5 to 1.5 hours.

In a preferred embodiment, the reactants are heated to about 90° to about 120°C. for about 1 hour at ambient pressure, cooled and then subjected to a vacuum of 13.3 to 133 kPa (10 to 100 mm Hg) for 30 to 60 minutes to remove water, ammonia and excess ethylene glycol. Sufficient volatile materials should be removed overhead so as to provide a liquid product containing from about 80 to about 95 wt.% of the initial charge and should have a water content of about 0.1 to about 2 wt.% and contain from about 6 wt.% to about 20 wt.% of molybdenum.

The resultant thus formed final ethylene glycol solution of a complex of ethylene glycol with ammonium-containing molybdenum compounds can then be recycled to the epoxidation reaction zone as the final ethylene glycol solution of a complex of ethylene glycol with ammonium-containing molybdenum compounds derived from a precipitate of solid ammonium-containing molybdenum compounds. Preferably, the catalyst mixture will contain from about 40 to about 60 wt.% of the initial ethylene glycol solution and, correspondingly, from about 60 to about 40 wt.% of the final ethylene glycol solution.

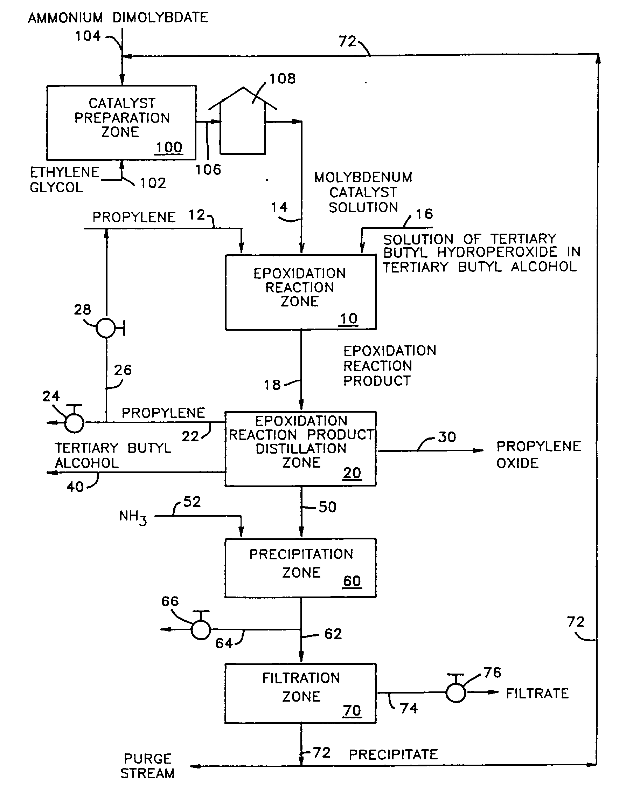

In the accompanying drawing, the figure is a schematic drawing of a preferred reaction and purification sequence that may be used in the practice of the present invention. In accordance with the present invention, a catalyst preparation zone 100 is provided to which substantially anhydrous ethylene glycol is charged by a line 102 and to which ammonium dimolybdate is charged by way of a line 104. The ethylene glycol and ammonium dimolybdate are charged in a manner such that the resultant mixture of ethylene glycol with ammonium dimolybdate will contain from about 7 to about 20 moles of ethylene glycol per gram atom of molybdenum in the ammonium dimolybdate and, more preferably, from about 8 to about 16 moles of ethylene glycol per gram atom of molybdenum contained in the ammonium dimolybdate.

The resultant mixture will normally contain water within the range of about 0.1 to about 2 wt.%.

In accordance with the present invention, the resultant mixture of ethylene glycol with ammonium dimolybdate is heated in the catalyst preparation zone which may suitably comprise an autoclave. The mixture is heated at a temperature between about 50° and about 150°C., and more preferably from 90° to 120°C., preferably at 0.1MPa (atmospheric pressure) for a period of time within the range of about 0.2 to about 2 hours, and more preferably 0.5 to about 1.5 hours in order to react the ammonium dimolybdate with some of the ethylene glycol present in the reaction mixture to form an ethylene glycol solution of an ethylene glycol-ammonium dimolybdate complex. Thereafter, the reaction mixture is cooled and then subjected to a vacuum of 13.3 to 133 kPa (10 to about 100 mm Hg) for 30 to 60 minutes while raising the temperature of the reaction medium to about 90° to 110°C. to provide a liquid bottoms product comprising about 80 to about 95 wt.% of the total initial charge weight and having a water content of about 0.1 to about 2 wt.%. This resultant solution of an ethylene glycol/ammonium dimolybdate complex in ethylene glycol will normally contain 6 to about 20 wt.% of molybdenum.

The catalyst preparation zone may suitably be operated as a batch operation and the desired initial ethylene glycol solution may be discharged from the catalyst preparation zone by a line 106 leading to a storage tank 108 from which the ethylene glycol solution may be charged to a reaction zone 10 by a molybdenum catalyst charge line 14.

Suitably, the tertiary butyl hydroperoxide that is charged to the epoxidation reaction zone 10 by way of line 16 is about a 40 to about 75 wt.% solution of tertiary butyl hydroperoxide in tertiary butyl alcohol. The catalyst is charged to the epoxidation reaction zone 10 by the charge line 14 in an amount such as to provide from about 50 to about 1000 ppm of molybdenum, based on the total of the reactants charged and, more preferably, from about 200 to 600 ppm. The epoxidation reaction is preferably conducted at superatmospheric pressure such as a pressure of about 2.1 to 7 MPa (300 to 1000 psig).

Propylene is charged by way of a line 12 to the epoxidation reaction zone 10 in an amount sufficient to provide an initial charge ratio of propylene to tertiary butyl hydroperoxide within the range of about 1.05:1 to about 2:1, and more preferably in the range of about 1.05:1 to about 1.8:1, and still more preferably in the range of about 1.05:1 to about 1.35:1.

When the reaction is conducted on a continuous basis, as illustrated in the drawing, the feed materials are charged to the epoxidation reaction zone 10 through the lines 12, 14 and 16 at rates sufficient to maintain the desired concentration of reactants and an equivalent volume of epoxidation reaction mixture is withdrawn from the epoxidation reaction zone 10 by way of a discharge line 18. The reaction product discharged by the line 18 will normally comprise a minor amount of unreacted propylene, tertiary butyl hydroperoxide, propylene oxide, tertiary butyl alcohol, including tertiary butyl alcohol formed by the reaction of the tertiary butyl hydroperoxide with propylene, the molybdenum catalyst and impurities such as propane, propionaldehyde, acetone, methanol, isopropanol, water, acetaldehyde, methyl formate, acetic acid, formic acid, isobutyric acid, esters, and hydrocarbons containing 6 or more carbon atoms and high boiling residue components.

The reaction product 18 is charged to an epoxidation reaction product distillation zone 20 where it is separated by distillation into desired fractions in accordance with methods known to those skilled in the art. For example, the distillation sequence disclosed in British Patent No. 1,298,253 may be used.

One of the distillate products that is recovered in the zone 20 is a propylene fraction which is discharged by a line 22 controlled by a valve 24 and provided with a branch line 26 controlled by a valve 28 in order to permit the recycle of unreacted propylene to the epoxidation reaction zone 10 through the propylene charge line 12.

Another distillate fraction that is obtained is a propylene oxide product fraction 30 which is discharged by the line 30.

The propylene oxide fraction may be purified in a propylene oxide purification zone (not shown) by known techniques such as, for example, those disclosed in Burnes et al. U. S. Patent No. 3,715,284, Schmidt et al. U. S. Patent No. 3,909,366, Schmidt U. S. Patent No. 3,881,996, Jubin U. S. Patent No. 3,607,669, Schmidt U. S. Patent No. 3,843,488 or Schmidt U. S. Patent No. 4,140,588.

Another product that is recovered from the epoxidation reaction product distillation zone 20 is a tertiary butyl alcohol distillate product 40 which may be further purified, if desired, to remove oxygenated impurities therefrom by catalytic treatment as disclosed, for example, in Sanderson et al. U. S. Patent No. 4,704,482, Sanderson et al. U. S. Patent No. 4,705,903 or Sanderson et al. U. S. Patent No. 4,742,149.

A heavy distillation fraction 50, usually a bottoms fraction, is also discharged from the epoxidation reaction product distillation zone 20. As described by Levine U. S. Patent No. 3,819,663 and Sweed U. S. Patent No. 4,455,283, the heavy distillation fraction will contain substantially all of the molybdenum catalyst initially charged to the epoxidation reaction zone 10 by way of the line 14. The heavy distillation fraction 50 will contain other products such as tertiary butyl hydroperoxide, tertiary butyl alcohol and impurities including oxygenates lighter than tertiary butyl alcohol such as acetaldehyde, acetone, isopropyl alcohol, etc., oxygenates heavier than tertiary butyl alcohol but lighter than tertiary butyl hydroperoxide, and residue components heavier than tertiary butyl hydroperoxide such as propylene glycol tertiary butyl ethers, etc. As indicated, the heavy distillation fraction 50 will also contain carboxylic acids such as formic acid, acetic acid and isobutyric acid and esters.

Although the molybdenum catalyst is present in the epoxidation reaction zone 10 in an amount in the range of about 50 to 1,000 ppm, and usually 200 to 600 ppm, it is progressively concentrated in the epoxidation reaction product distillation zone 20 and is normally present in the heavy distillation fraction 50 in an amount in the range of about 0.4 to 0.8 wt.% (about 4,000 to 8,000 ppm).

The molybdenum-contaminated heavy distillation fraction 50, in accordance with the present invention, is charged to a precipitation zone 60 which may comprise a reaction vessel such as an autoclave which is equipped with suitably agitation means (e.g., an impeller) and suitably temperature control means such as an external jacket or internal coils through which a heat exchange medium can be circulated. Within the precipitation zone the heavy distillation fraction 50 is brought into contact with ammonia which is charged by an ammonia charge line 52 in at least an equimolar amount, based on the molybdenum content of the heavy distillation fraction 50 and, preferably, in a molar excess. The ammonia is preferably used in the form of anhydrous ammonia in order to minimize the water content of the treated heavy distillation fraction 50. The ammonia is suitably brought into contact with the heavy distillation fraction 50 under ambient temperature and pressure conditions, although higher temperatures and/or pressures may be used, such as temperatures within the range of about 20° to 250°C. and pressures within the range of 0.1 to 20.8 MPa (0 to about 3,000 psig). The contact time should be sufficient to ensure as complete a reaction of the ammonia with the molybdenum as is reasonably possible and to ensure substantially complete precipitation of the product, such as a contact time of about 0.2 to 2 hours.

The thus-formed slurry of precipitate in the treated heavy distillation fraction 50 is discharged from the precipitation zone 60 by a slurry discharge line 62 leading to a precipitate separating zone, such as a filtration zone 70 where the slurry is resolved into a precipitate that is removed by a discharge line 72 and a substantially molybdenum-free filtrate fraction that is discharged by a filtrate discharge line 74 controlled by a valve 76.

The precipitate 72 is then charged to catalyst preparation zone 100 together with ethylene glycol, preferably anhydrous, charged by a line 102. The resultant final catalyst solution is discharged from the catalyst regeneration zone 80 by a line 106 leading to the junction 112 where it is mixed with the initial ethylene glycol solution to provide the catalyst reaction mixture discharge from the junction 112 by the line 14 leading to the epoxidation reaction zone 10.

The invention will be further described with reference to the following non-limiting Examples.

### Preparation of Final Catalyst Solution

A series of catalyst compositions (final ethylene glycol solution) were prepared by reacting ethylene glycol with the molybdenum precipitate produced by ammonia treatment of the heavy distillation fraction from epoxidation.

### Example 1

About 4.8 g of an ammonium molybdate-containing precipitate containing about 48.4 % molybdenum were charged to a 250 ml flask together with 15.0 g of substantially anhydrous ethylene glycol. The flask was equipped with a magnetic stirrer, a K-head, a condenser and a nitrogen purge. The flask was heated to 100°C. and held at this temperature for about 1 hour. Thereafter the contents of the flask were cooled and then subjected to a vacuum to take overhead about 1.3 g of material, leaving a total of 16.5 g of material in the bottom of the flask.

Since the total charge to the flask was 19.8 g, the bottoms resulting after vacuum evacuation amounted to about 83% of the charge. On analysis it was found that a total of 2.323 g of molybdenum were charged and that the molybdenum in the bottoms resulting from vacuum distillation amounted to 2.31 g indicating that 99.4 wt.% of the charged molybdenum was incorporated into the catalyst. The final bottoms was found by analysis to contain 3.17 wt.% of water and the analysis was 14.0 wt.% molybdenum, acid number 160.78, nitrogen (micro Kjeldahl) 0.84.

### Example 2

About 6.2 g of an ammonia precipitate containing about 48.0 % molybdenum were charged to a 250 ml flask together with 19.2 g of substantially anhydrous ethylene glycol. The flask was equipped with a magnetic stirrer, a K-head, a condenser and a nitrogen purge. The flask was heated to 100°C. and held at this temperature for about 1 hour. Thereafter the contents of the flask were cooled and then subjected to a vacuum to take overhead about 1.7 g of material, leaving a total of 21.2 g of material in the bottom of the flask.

Since the total charge to the flask was 25.4 g, the bottoms resulting after vacuum evacuation amounted to about 83.1% of the charge. On analysis it was found that a total of 2.976 g of molybdenum were charged and that the molybdenum in the bottoms resulting from vacuum distillation amounted to 3.307, indicating an analytical error, because this would indicate that 111% of the charged molybdenum was incorporated into the catalyst. The final bottoms was found by analysis to contain 1.84 wt.% of water and the analysis was 15.6 wt.% molybdenum, acid number 169.36, nitrogen (micro Kjeldahl) 0.72. The analytical error is likely in the Atomic Absorption determination of molybdenum in the catalyst bottoms. The maximum amount of molybdenum that could be in the bottoms (basis the 2.976 g charged) would be 14.0% molybdenum.

### Example 3

About 5.0 g of an ammonia precipitate containing about 46.0 % molybdenum were charged to a 250 ml flask together with 14.72 g of substantially anhydrous ethylene glycol. The flask was equipped with a magnetic stirrer, a K-head, a condenser and a nitrogen purge. The flask was heated to 100°C. and held at this temperature for about 1 hour. Thereafter the contents of the flask were cooled and then subjected to a vacuum to take overhead about 2.4 g of material, leaving a total of 15.1 g of material in the bottom of the flask.

Since the total charge to the flask was 19.72 g, the bottoms resulting after vacuum evacuation amounted to about 76.6 wt.% of the charge. On analysis it was found that a total of 2.300 g of molybdenum were charged and that the molybdenum in the bottoms resulting from vacuum distillation amounted to 2.356, indicating a small analytical error, because this would indicate that 102.4 wt.% of the charged molybdenum was incorporated into the catalyst. The final bottoms was found by analysis to contain 1.53 wt.% of water and the analysis was 15.6 wt.% molybdenum, acid number 180.54. The maximum amount of molybdenum that could be in the bottoms (basis 2.300 g molybdenum charged) would be 15.23%.

### Example 4

About 4.4 g of an ammonia precipitate containing about 5.0 % molybdenum were charged to a 250 ml flask together with 14.0 g of substantially anhydrous ethylene glycol. The flask was equipped with a magnetic stirrer, a K-head, a condenser and a nitrogen purge. The flask was heated to 100°C. and held at this temperature for about 1 hour. Thereafter the contents of the flask were cooled and then subjected to a vacuum to take overhead about 1.7 g of material, leaving a total of 14.8 g of material in the bottom of the flask.

Since the total charge to the flask was 18.8 g, the bottoms resulting after vacuum evacuation amounted to about 78.7 wt.% of the charge. On analysis it was found that a total of 2.200 g of molybdenum were charged and that the molybdenum in the bottoms resulting from vacuum distillation amounted to 2.2644 g, indicating a small analytical error, because this would indicate that about 103% of the charged molybdenum was incorporated into the catalyst. The final bottoms was found by analysis to contain 0.04 wt.% of water and the analysis was 15.3 wt.% molybdenum, acid number 162.14, nitrogen (micro Kjeldahl) 0.655. The percent molybdenum in the bottoms (basis 2.20 g molybdenum charged) could be a maximum of 14.86%.

### Example 5

About 6.0 g of an ammonia precipitate containing about 47.0 % molybdenum were charged to a 250 ml flask together with 18.33 g of substantially anhydrous ethylene glycol. The flask was equipped with a magnetic stirrer, a K-head, a condenser and a nitrogen purge. The flask was heated to 100°C. and held at this temperature for about 1 hour. Thereafter the contents of the flask were cooled and then subjected to a vacuum to take overhead about 2.9 g of material, leaving a total of 18.9 g of material in the bottom of the flask.

Since the total charge to the flask was 24.33 g, the bottoms resulting after vacuum evacuation amounted to about 77.7% of the charge. On analysis it was found that a total of 2.820 g of molybdenum were charged and that the molybdenum in the bottoms resulting from vacuum distillation amounted to 2.927, indicating a small analytical error, because this would indicate that 105% of the charged molybdenum was incorporated into the catalyst. The final bottoms was found by analysis to contain 0.11 wt.% of water and the analysis was 15.7 wt.% molybdenum, acid number 173.13, nitrogen (micro Kjeldahl) 1.02. The maximum percent molybdenum that could be in the bottoms would be 14.92% (basis the 2.82 g molybdenum charged).

The results of the foregoing catalyst preparation experiments are summarized in Table I.

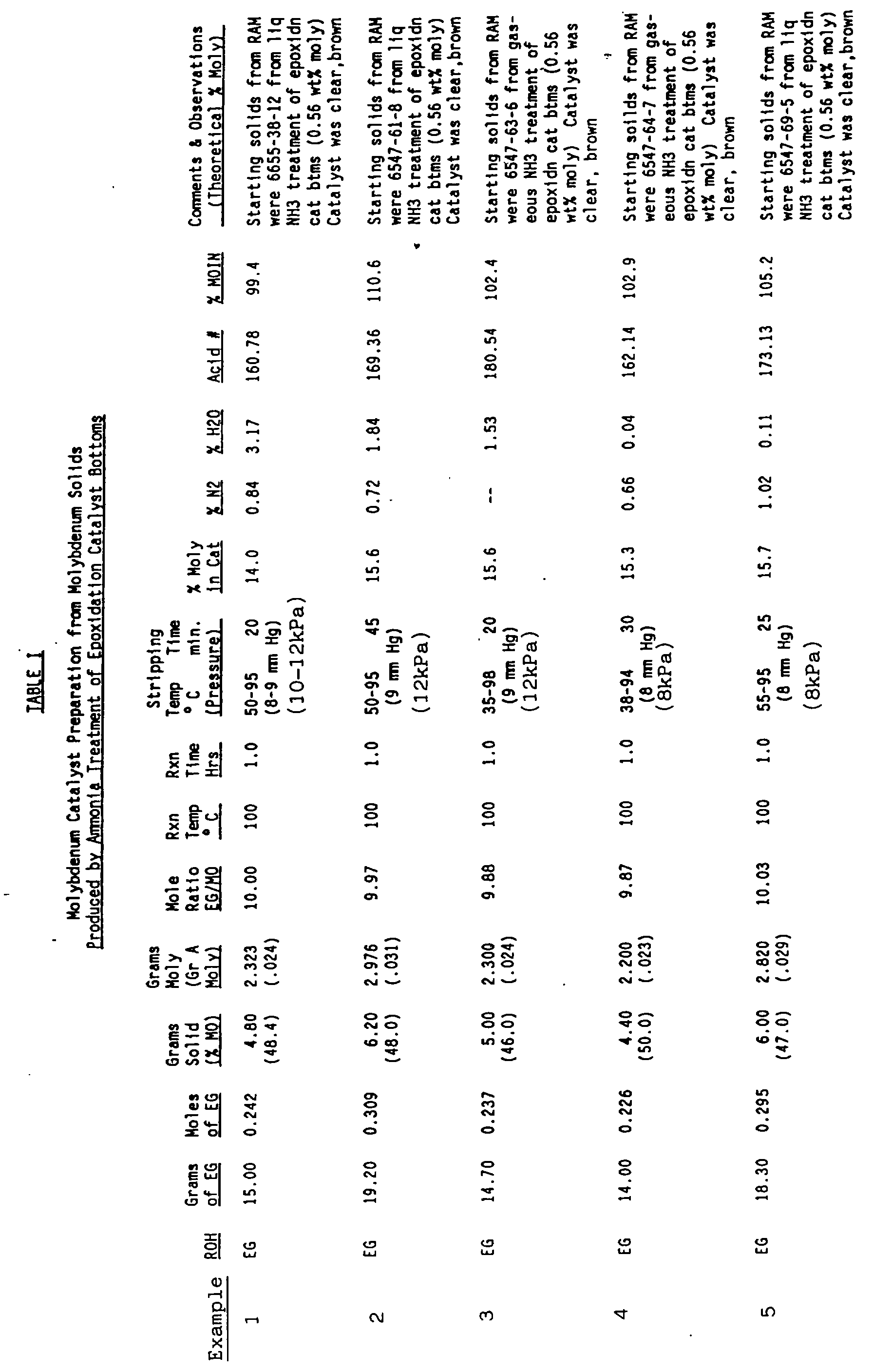

### Epoxidation Examples

In the following examples, the catalyst complexes of Examples 1-5 were used to catalyze the reaction of octene-1 with tertiary butyl hydroperoxide. Octene-1 was used rather than propylene for convenience in the laboratory and in order to more conveniently obtain an evaluation of the effectiveness of the catalyst of Examples 1-5. The epoxidation reaction can be run at atmospheric pressure in laboratory glassware when the olefin is octene-1.

The epoxidation results are summarized in attached Table II.

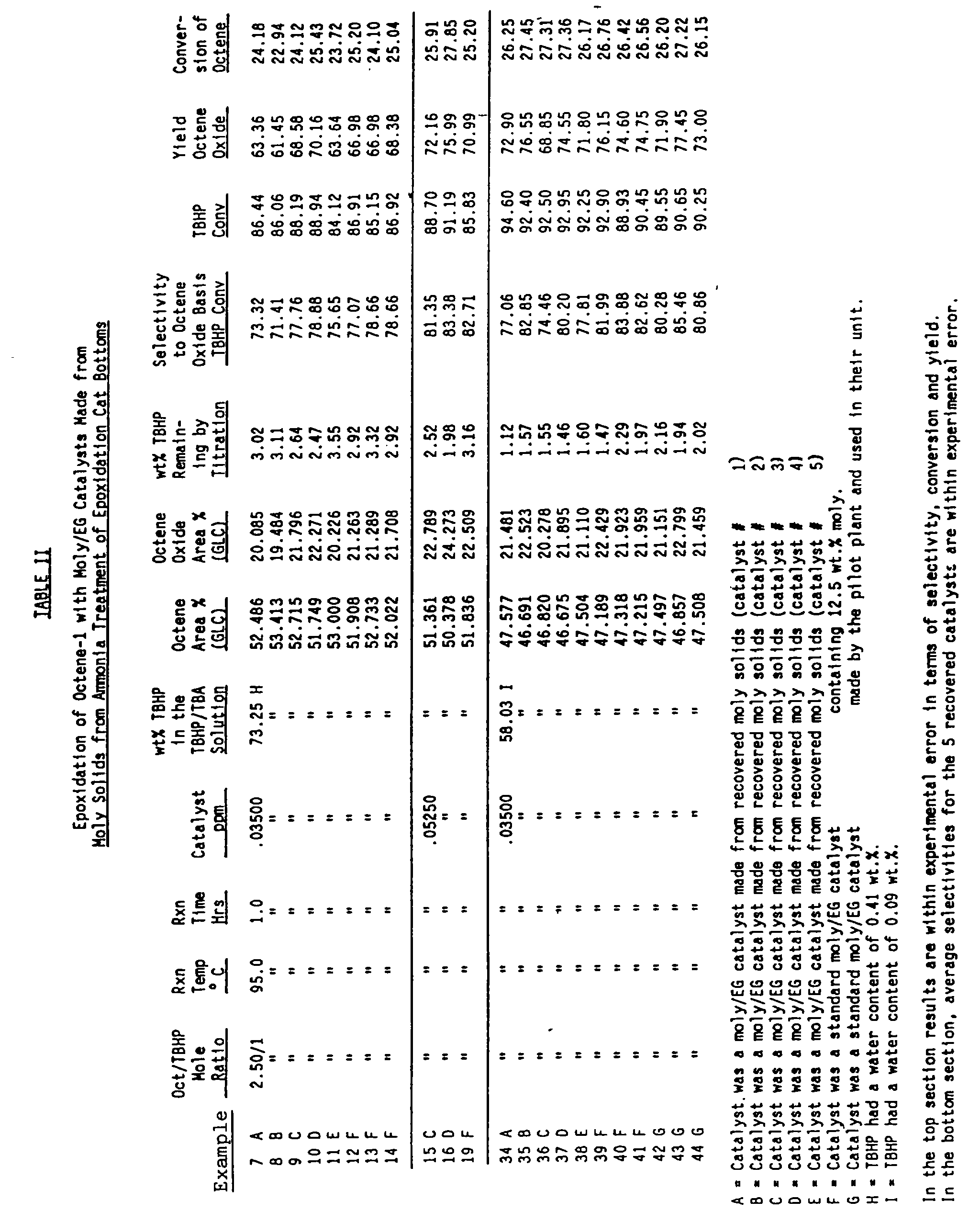

A statistical analysis was made of the results reported in Table II. From the statistical analysis it was concluded that:
A) The difference in means for selectivity to octene oxide is statistically significant at the 95-98% probability level for virgin and recycled catalyst. The selectivity is higher with virgin catalyst.
B) The difference in means is not statistically significant for TBHP conversion, octene oxide yield and octene conversion at the 95-98% probability level.

## Claims

1. A method for the preparation of tertiary butyl alcohol and C₃-C₁₂ linear olefin mono-epoxide comprising
a) reacting C₃-C₁₂ linear alpha mono olefin with tertiary butyl hydroperoxide in solution in tertiary butyl alcohol in the presence of a soluble complex of molybdenum with ethylene glycol to provide an epoxidation reaction product,
b) fractionating the reaction product to provide a heavy liquid distillation fraction comprising dissolved molybdenum-ethylene glycol catalyst complex, characterised by,
c) saturating the heavy distillation fraction with ammonia in a precipitation zone to provide a solid ammonium-containing molybdenum precipitate
d) recovering and reacting said precipitate with substantially anhydrous ethylene glycol to form an ethylene glycol solution of a complex of ethylene glycol with ammonium-containing compounds present in the precipitate,
e) heating said ethylene glycol solution in a catalyst regeneration zone at a pressure of 0.1 to 20.8 MPa (0 to 3000 psig) and a reaction temperature of 70° to 250°C to form a substantially solids-free ethylene glycol solution of a complex of ethylene glycol with the ammonium-containing compounds in said solid ammonium-containing molybdenum precipitate,
f) holding said solids-free solution of said complex of ethylene glycol with the said ammonium-containing compounds in said catalyst regeneration zone at a temperature of 90° to 110°C to permit vaporization and removal of volatile by-products, including water, by an amount sufficient to provide an ethylene glycol solution containing 17 to 52 wt.% of said complex of ethylene glycol with said ammonium-containing molybdenum compounds and having a molybdenum content of 6 to 20 wt.% and a water concentration of 0.5 to 6 wt.% and
g) recycling said solution for use as a catalyst in the above reaction of linear mono-olefin and tertiary butyl hydroperoxide.

2. A method as claimed in Claim 1 wherein the recovered solid ammonium-containing molybdenum precipitate is charged to a catalyst regeneration zone and mixed with an amount of substantially anhydrous ethylene glycol sufficient to provide from 7 to 20 moles of ethylene glycol per gram atom of molybdenum contained in said solid ammonium-containing molybdenum precipitate to form an ethylene glycol feed mixture, said mixture being heated in said catalyst regeneration zone at a pressure of 0.1 to 20.8 MPa (0 to 3000 psig) and a reaction temperature of 70°C to 250°C for a time of 1 to 2 hours to form a substantially solids-free ethylene glycol solution of a complex of ethylene glycol with the ammonium-containing compounds in said solid ammonium-containing molybdenum precipitate, said solution being held in said catalyst regeneration zone at a temperature of 90°C to 110°C for from 0.5 to 5 hours in order to vaporize and remove volatile by-products to provide an ethylene glycol solution containing 17 to 52 wt.% of said complex of ethylene glycol with said ammonium-containing molybdenum compounds and having a molybdenum content of 6 to 20 wt.% and a water concentration of 0.5 to 6 wt.%.

3. A method as claimed in Claim 1 or Claim 2 wherein the alpha olefin is propylene.

4. A method as claimed in any one of Claims 1 to 3 wherein the heavy liquid distillation fraction is saturated with ammonia in the said precipitation zone under precipitation conditions including a temperature of about 20°C to about 250°C and a pressure of 0.1 to 20.8 MPa (0 to 3000 psig).

5. A method as claimed in any one of Claims 1 to 4 wherein the heavy liquid distillation fraction contains from 0.4 to 0.8 wt.% of molybdenum, the said filtrate contains 50 to 200 ppm of molybdenum and the said precipitated ammonium-containing molybdenum compounds contain 40 to about 50 wt.% of molybdenum.

6. A continuous method for the molybdenum-catalyzed preparation of tertiary butyl alcohol and propylene oxide from propylene and tertiary butyl hydroperoxide which comprises the steps of:
a. continuously charging substantially anhydrous ethylene glycol and substantially anhydrous ammonium dimolybdate to a catalyst preparation reactor and mixing them therein in amounts sufficient to provide 7 to 20 moles of ethylene glycol per gram atom of molybdenum contained in said ammonium dimolybdate,
b. continuously heating said mixture of ethylene glycol and ammonium dimolybdate in said catalyst preparation reactor at a pressure of 0.1 to 20.8 MPa (0 to 3000 psig) and a reaction temperature of 70°C to 250°C for from 1 to 2 hours sufficient to form a solids-free solution of a complex of ethylene glycol with the said ammonium dimolybdate,
c. continuously holding said solids-free solution of said complex of ethylene glycol with ammonium dimolybdate in said catalyst preparation reactor at a temperature of 90°C to 110°C for from 0.5 to 5 hours, sufficient to permit vaporization and removal of volatile by-products, including water, by an amount sufficient to provide an initial ethylene glycol solution containing 16 to 52 wt.% of said complex of ethylene glycol with said ammonium dimolybdate and having a molybdenum content of 6 to 20 wt.% and a water concentration of 0.5 to 6 wt.%,
d. continuously charging a tertiary butyl alcohol solution of propylene and tertiary butyl hydroperoxide to an epoxidation reaction zone together with a catalytic amount of a catalyst mixture of (aa) said initial ethylene glycol solution of said complex of ethylene glycol with said ammonium dimolybdate with (bb) a final ethylene glycol solution of a complex of ethylene glycol with ammonium-containing molybdenum compounds derived from a precipitate of solid ammonium containing molybdenum compounds, in amounts sufficient to provide an initial molar ratio of propylene-to tertiary butyl hydroperoxide of 1.05:1 to 2:1 and a concentration of 100 to 600 ppm of said catalyst mixture in said tertiary butyl alcohol solution,
e. establishing epoxidation reaction conditions in said epoxidation reaction zone including a temperature of 50°C to 180°C and a reaction time of 1 to 5 hours to thereby react said propylene with said tertiary butyl hydroperoxide and form an epoxidation reaction product comprising unreacted propylene, unreacted tertiary butyl hydroperoxide, propylene oxide, tertiary butyl alcohol, dissolved molybdenum-ethylene glycol catalyst complex, and oxygen-containing impurities,
f. continuously resolving the said epoxidation reaction product in a distillation zone into distillation fractions including a distillate propylene fraction, a distillate propylene oxide fraction, a distillate tertiary butyl alcohol fraction and a heavy liquid distillation fraction composed primarily of tertiary butyl hydroperoxide, tertiary butyl alcohol, oxygen-containing impurities and dissolved molybdenum-ethylene glycol catalyst complex,
g. continuously charging said heavy distillation fraction to a precipitation zone and saturating said heavy distillation fraction in said precipitation zone with ammonia to thereby form a liquid amination product containing a precipitate of solid ammonium-containing molybdenum compounds,
h. continuously charging said amination product to a separation zone and therein separating the solid ammonium-containing molybdenum precipitate therefrom,
i. continuously recovering said solid ammonium-containing molybdenum precipitate,
j. continuously charging said recovered solid ammonium-containing molybdenum precipitate to a catalyst regeneration zone and mixing it therein with an amount of substantially anhydrous ethylene glycol sufficient to provide 7 to 20 moles of ethylene glycol per gram atom of molybdenum contained in said solid ammonium-containing molybdenum precipitate to form an ethylene glycol feed mixture,
k. continuously heating said ethylene glycol feed mixture in said catalyst regeneration zone at a pressure of 0.1 to 20.8 MPa (O to 3000 psig) and a reaction temperature of 70°C to 250°C for from 1 to 2 hours sufficient to form a solids-free solution of a complex of ethylene glycol with the ammonium-containing compounds in said solid ammonium-containing molybdenum precipitate,
1. holding said solids-free solution of said complex of ethylene glycol with the said ammonium-containing compounds in said catalyst regeneration zone at a temperature of 90°C to 110°C for from 0.5 to 5 hours, sufficient to permit vaporization and removal of volatile by-products, including water, by an amount sufficient to provide a final ethylene glycol solution containing 16 to 52 wt.% of said complex ethylene glycol with said ammonium-containing molybdenum compounds and having a molybdenum content of 6 to 20 wt.% and a water concentration of 0.5 to 6 wt.%, and
m. continuously charging said thus-prepared final ethylene glycol solution of a complex of ethylene glycol with ammonium-containing molybdenum compounds to said epoxidation reaction zone as said final ethylene glycol solution of a complex of ethylene glycol with ammonium-containing molybdenum compounds derived from a precipitate of solid ammonium containing molybdenum compounds.

## Patentansprüche

1. Ein Verfahren zur Herstellung von tert.-Butylalkohol und linearem C₃-C₁₂-Olefinmonoepoxid, welches umfaßt, daß
a) lineares C₃-C₁₂-alpha-Monoolefin mit tert.-Butylhydroperoxid in Lösung in tert.-Butylalkohol in der Gegenwart eines löslichen Komplexes von Molybdän mit Ethylenglykol zur Reaktion gebracht wird, um ein Epoxidationsreaktionsprodukt bereitzustellen,
b) das Reaktionsprodukt fraktioniert wird, um eine schwere flüssige Destillationsfraktion bereitzustellen, die gelösten Molybdän-Ethylenglykol-Katalysatorkomplex umfaßt,
dadurch gekennzeichnet, daß
c) die schwere Destillationsfraktion in einer Fällungszone mit Ammoniak gesättigt wird, um einen festen ammoniumhaltigen Molybdän-Niederschlag bereitzustellen,
d) besagter Niederschlag gewonnen und mit im wesentlichen wasserfreiem Ethylenglykol zur Reaktion gebracht wird, um eine Ethylenglykollösung eines Komplexes von Ethylenglykol mit ammoniumhaltigen Verbindungen, die im Niederschlag vorliegen, zu bilden,
e) besagte Ethylenglykollösung in einer Katalysatorregenerationszone bei einem Druck von 0,1 bis 20,8 MPa (0 bis 3.000 psig) und einer Reaktionstemperatur von 70° bis 250°C erhitzt wird, um eine im wesentlichen von Feststoffen freie Ethylenglykollösung eines Komplexes von Ethylenglykol mit den ammoniumhaltigen Verbindungen in besagtem festen ammoniumhaltigen Molybdän-Niederschlag zu bilden,
f) besagte von Feststoffen freie Lösung besagten Komplexes von Ethylenglykol mit den besagten ammoniumhaltigen Verbindungen in besagter Katalysatorregenerationszone bei einer Temperatur von 90° bis 110°C gehalten wird, um Verdampfung und Entfernung von flüchtigen Nebenprodukten, einschließlich Wasser, um eine Menge zu ermöglichen, die ausreichend ist, um eine Ethylenglykollösung bereitzustellen, die 17 bis 52 Gew.-% besagten Komplexes von Ethylenglykol mit besagten ammoniumhaltigen Molybdänverbindungen enthält und einen Molybdängehalt von 6 bis 20 Gew.-% und eine Wasserkonzentration von 0,5 bis 6 Gew.-% aufweist, und
g) besagte Lösung zur Verwendung als ein Katalysator in der obigen Reaktion von linearem Monoolefin und tert.-Butylhydroperoxid rückgeführt wird.

2. Ein Verfahren nach Anspruch 1, wobei der gewonnene feste ammoniumhaltige Molybdän-Niederschlag einer Katalysatorregenerationszone zugeführt und mit einer Menge von im wesentlichen wasserfreiem Ethylenglykol vermischt wird, die ausreichend ist, um von 7 bis 20 Mol Ethylenglykol pro Grammatom Molybdän, das in besagtem festen ammoniumhaltigen Molybdän-Niederschlag enthalten ist, bereitzustellen, um eine Ethylenglykol-Chargenmischung zu bilden, wobei besagte Mischung in besagter Katalysatorregenerationszone bei einem Druck von 0,1 bis 20,8 MPa (0 bis 3.000 psig) und einer Reaktionstemperatur von 70°C bis 250°C für eine Zeit von 1 bis 2 Stunden erhitzt wird, um eine im wesentlichen von Feststoff freie Ethylenglykollösung eines Komplexes von Ethylenglykol mit den ammoniumhaltigen Verbindungen in besagtem festen ammoniumhaltigen Molybdän-Niederschlag zu bilden, wobei besagte Lösung in besagter Katalysatorregenerationszone bei einer Temperatur von 90°C bis 110°C für von 0,5 bis 5 Stunden gehalten wird, um flüchtige Nebenprodukte zu verdampfen und zu entfernen, um eine Ethylenglykollösung bereitzustellen, die 17 bis 52 Gew.-% besagten Komplexes von Ethylenglykol mit besagten ammoniumhaltigen Molybdänverbindungen enthält und einen Molybdängehalt von 6 bis 20 Gew.-% und eine Wasserkonzentration von 0,5 bis 6 Gew.-% aufweist.

3. Ein Verfahren nach Anspruch 1 oder Anspruch 2, wobei das alpha-Olefin Propylen ist.

4. Ein Verfahren nach einem der Ansprüche 1 bis 3, wobei die schwere flüssige Destillationsfraktion in besagter Fällungszone unter Fällungsbedingungen, einschließlich einer Temperatur von etwa 20°C bis etwa 250°C und einem Druck von 0,1 bis 20,8 MPa (0 bis 3.000 psig), mit Ammoniak gesättigt wird.

5. Ein Verfahren nach einem der Ansprüche 1 bis 4, wobei die schwere flüssige Destillationsfraktion von 0,4 bis 0,8 Gew.-% Molybdän enthält, das besagte Filtrat 50 bis 200 ppm Molybdän enthält und die besagten gefällten ammoniumhaltigen Molybdänverbindungen 40 bis etwa 50 Gew.-% Molybdän enthalten.

6. Ein kontinuierliches Verfahren für die durch Molybdän katalysierte Herstellung von tert.-Butylalkohol und Propylenoxid aus Propylen und tert.-Butylhydroperoxid, welches die Schritte umfaßt, daß:
a. im wesentlichen wasserfreies Ethylenglykol und im wesentlichen wasserfreies Ammoniumdimolybdat kontinuierlich einem Katalysatorherstellungsreaktor zugeführt und sie darin vermischt werden, in Mengen, die ausreichend sind, um 7 bis 20 Mol Ethylenglykol pro Grammatom Molybdän, das in besagtem Ammoniumdimolybdat enthalten ist, bereitzustellen,
b. besagte Mischung von Ethylenglykol und Ammoniumdimolybdat in besagtem Katalysatorherstellungsreaktor bei einem Druck von 0,1 bis 20,8 MPa (0 bis 3.000 psig) und einer Reaktionstemperatur von 70°C bis 250°C für von 1 bis 2 Stunden kontinuierlich erhitzt wird, was ausreichend ist, um eine von Feststoffen freie Lösung eines Komplexes von Ethylenglykol mit dem besagten Ammoniumdimolybdat zu bilden,
c. besagte von Feststoffen freie Lösung besagten Komplexes von Ethylenglykol mit Ammoniumdimolybdat kontinuierlich in besagtem Katalysatorherstellungsreaktor bei einer Temperatur von 90°C bis 110°C für von 0,5 bis 5 Stunden gehalten wird, was ausreichend ist, um Verdampfung und Entfernung von flüchtigen Nebenprodukten, einschließlich Wasser, um eine Menge zu ermöglichen, die ausreichend ist, um eine Anfangs-Ethylenglykollösung bereitzustellen, die 16 bis 52 Gew.-% besagten Komplexes von Ethylenglykol mit besagtem Ammoniumdimolybdat enthält und einen Molybdängehalt von 6 bis 20 Gew.-% und eine Wasserkonzentration von 0,5 bis 6 Gew.-% aufweist,
d. eine tert.-Butylalkohollösung von Propylen und tert.-Butylhydroperoxid zusammen mit einer katalytischen Menge einer Katalysatormischung aus (aa) besagter Anfangs-Ethylenglykollösung besagten Komplexes von Ethylenglykol mit besagtem Ammoniumdimolybdat mit (bb) einer End-Ethylenglykollösung eines Komplexes von Ethylenglykol mit ammoniumhaltigen Molybdänverbindungen, die aus einem Niederschlag von festen ammoniumhaltigen Molybdänverbindungen stammen, einer Epoxidationsreaktionszone kontinuierlich in Mengen zugeführt werden, die ausreichend sind, um ein Anfangs-Molverhältnis von Propylen zu tert.-Butylhydroperoxid von 1,05:1 bis 2:1 und eine Konzentration von 100 bis 600 ppm von besagter Katalysatormischung in besagter tert.-Butylalkohollösung bereitzustellen,
e. Epoxidationsreaktionsbedingungen in besagter Epoxidationsreaktionszone aufgebaut werden, einschließlich einer Temperatur von 50°C bis 180°C und einer Reaktionszeit von 1 bis 5 Stunden, um dadurch besagtes Propylen mit besagtem tert.-Butylhydroperoxid zur Reaktion zu bringen und ein Epoxidationsreaktionsprodukt zu bilden, das nicht-umgesetztes Propylen, nicht-umgesetztes tert.-Butylhydroperoxid, Propylenoxid, tert.-Butylalkohol, gelösten Molybdän-Ethylenglykol-Katalysatorkomplex und sauerstoffhaltige Verunreinigungen umfaßt,
f. das besagte Epoxidationsreaktionsprodukt in einer Destillationszone kontinuierlich in Destillationsfraktionen aufgetrennt wird, einschließlich einer Destillat-Propylenfraktion, einer Destillat-Propylenoxidfraktion, einer Destillat-tert.-Butylalkoholfraktion und einer schweren flüssigen Destillationsfraktion, die primär aus tert.-Butylhydroperoxid, tert.-Butylalkohol, sauerstoffhaltigen Verunreinigungen und gelöstem Molybdän-Ethylenglykol-Katalysatorkomplex besteht,
g. besagte schwere Destillationsfraktion kontinuierlich einer Fällungszone zugeführt und besagte schwere Destillationsfraktion in besagter Fällungszone mit Ammoniak gesättigt wird, um dadurch ein flüssiges Aminierungsprodukt zu bilden, das einen Niederschlag von festen ammoniumhaltigen Molybdänverbindungen enthält,
h. besagtes Aminierungprodukt kontinuierlich einer Trennzone zugeführt und darin der feste ammoniumhaltige Molybdän-Niederschlag davon abgetrennt wird,
i. besagter fester ammoniumhaltiger Molybdän-Niederschlag kontinuierlich gewonnen wird,
j. besagter gewonnener fester ammoniumhaltiger Molybdän-Niederschlag kontinuierlich einer Katalysatorregenerationszone zugeführt und er darin mit einer Menge von im wesentlichen wasserfreiem Ethylenglykol vermischt wird, die ausreichend ist, um 7 bis 20 Mol Ethylenglykol pro Grammatom Molybdän, das in besagtem festen ammoniumhaltigen Molybdän-Niederschlag enthalten ist, bereitzustellen, um eine Ethylenglykol-Chargenmischung zu bilden,
k. besagte Ethylenglykol-Chargenmischung in besagter Katalysatorregenerationszone bei einem Druck von 0,1 bis 20,8 MPa (0 bis 3.000 psig) und einer Reaktionstemperatur von 70°C bis 250°C für von 1 bis 2 Stunden kontinuierlich erhitzt wird, was ausreichend ist, um eine von Feststoffen freie Lösung eines Komplexes von Ethylenglykol mit den ammoniumhaltigen Verbindungen in besagtem festen ammoniumhaltigen Molybdän-Niederschlag zu bilden,
l. besagte von Feststoffen freie Lösung besagten Komplexes von Ethylenglykol mit den besagten ammoniumhaltigen Verbindungen in besagter Katalysatorregenerationszone bei einer Temperatur von 90°C bis 110°C für von 0,5 bis 5 Stunden gehalten wird, was ausreichend ist, um Verdampfung und Entfernung von flüchtigen Nebenprodukten, einschließlich Wasser, um eine Menge zu ermöglichen, die ausreichend ist, um eine End-Ethylenglykollösung bereitzustellen, die 16 bis 52 Gew.-% von besagtem Komplex-Ethylenglykol mit besagten ammoniumhaltigen Molybdänverbindungen enthält und einen Molybdängehalt von 6 bis 20 Gew.-% und eine Wasserkonzentration von 0,5 bis 6 Gew.-% aufweist, und
m. besagte so hergestellte End-Ethylenglykollösung eines Komplexes von Ethylenglykol mit ammoniumhaltigen Molybdänverbindungen kontinuierlich zu besagter Epoxidationsreaktionszone als besagte End-Ethylenglykollösung eines Komplexes von Ethylenglykol mit ammoniumhaltigen Molybdänverbindungen, die aus einem Niederschlag von festen ammoniumhaltigen Molybdänverbindungen stammen, zugeführt wird.

## Revendications

1. Procédé pour la préparation d'alcool tertiobutylique et d'un mono-époxyde d'oléfine en C₃ à C₁₂ linéaire, comprenant
a) la réaction d'une alpha mono-oléfine en C₃ à C₁₂ linéaire avec de l'hydroperoxyde de tertiobutyle en solution dans de l'alcool tertiobutylique, en présence d'un complexe soluble de molybdène, avec de l'éthylèneglycol pour fournir un produit de réaction d'époxydation,
b) le fractionnement du produit de réaction pour fournir une fraction de distillation liquide lourde comprenant le complexe catalyseur molybdène-éthylèneglycol dissous, caractérisé par,
c) la saturation de la fraction de distillation lourde avec de l'ammoniac dans une zone de précipitation pour fournir un précipité solide de molybdène contenant de l'ammonium
d) la récupération et la réaction dudit précipité avec de l'éthylèneglycol substantiellement anhydre pour former une solution d'éthylèneglycol d'un complexe d'éthylèneglycol avec les composés contenant de l'ammonium présents dans le précipité,
e) le chauffage de ladite solution d'éthylèneglycol dans une zone de régénération de catalyseur à une pression de 0,1 à 20,8 MPa (0 à 3000 psig) et une température de réaction de 70 à 250 °C pour former une solution d'éthylèneglycol substantiellement exempte de solides d'un complexe d'éthylèneglycol avec les composés contenant de l'ammonium dans le précipité solide de molybdène contenant de l'ammonium,
f) le maintien de ladite solution exempte de solides, dudit complexe d'éthylèneglycol avec lesdits composés contenant de l'ammonium, dans ladite zone de régénération de catalyseur à une température de 90 à 110 °C pour permettre la vaporisation et l'élimination des sous-produits volatils, y compris l'eau, en une quantité suffisante pour fournir une solution d'éthylèneglycol contenant 17 à 52 % en poids dudit complexe d'éthylèneglycol avec lesdits composés de molybdène contenant de l'ammonium et ayant une teneur en molybdène de 6 à 20 % en poids et une concentration en eau de 0,5 à 6 % en poids et
g) le recyclage de ladite solution pour l'utilisation en tant que catalyseur dans la réaction ci-dessus d'une mono-oléfine linéaire et d'hydroperoxyde de tertiobutyle.

2. Procédé selon la revendication 1, dans lequel le précipité solide de molybdène contenant de l'ammonium récupéré est chargé dans une zone de régénération de catalyseur et mélangé avec une quantité d'éthylèneglycol substantiellement anhydre suffisante pour fournir de 7 à 20 moles d'éthylèneglycol par atome gramme de molybdène contenu dans ledit précipité solide de molybdène contenant de l'ammonium et former un mélange d'alimentation d'éthylèneglycol, ledit mélange étant chauffé dans ladite zone de régénération de catalyseur à une pression de 0,1 à 20,8 MPa (0 à 3000 psig) et une température de réaction de 70 °C à 250 °C pendant une durée de 1 à 2 h pour former une solution d'éthylèneglycol, substantiellement exempte de solides d'un complexe d'éthylèneglycol avec les composés contenant de l'ammonium dans le précipité solide de molybdène contenant de l'ammonium, ladite solution étant maintenue dans ladite zone de régénération de catalyseur à une température de 90 °C à 110 °C pendant de 0,5 à 5 h afin de vaporiser et éliminer les sous-produits volatils et fournir une solution d'éthylèneglycol contenant 17 à 52 % en poids dudit complexe d'éthylèneglycol avec lesdits composés de molybdène contenant de l'ammonium et ayant une teneur en molybdène de 6 à 20 % en poids et une concentration en eau de 0,5 à 6 % en poids.

3. Procédé selon la revendication 1 ou la revendication 2, dans lequel l'alpha-oléfine est le propylène.

4. Procédé selon l'une quelconque des revendications 1 à 3, dans lequel la fraction de distillation liquide lourde est saturée avec de l'ammoniac dans ladite zone de précipitation dans des conditions de précipitation incluant une température d'environ 20 °C à environ 250 °C et une pression de 0,1 à 20,8 MPa (0 à 3000 psig).

5. Procédé selon l'une quelconque des revendications 1 à 4, dans lequel la fraction de distillation liquide lourde contient de 0,4 à 0,8 % en poids de molybdène, ledit filtrat contient 50 à 200 ppm de molybdène et lesdits composés précipités de molybdène contenant de l'ammonium contiennent 40 à environ 50 % en poids de molybdène.

6. Procédé continu pour la préparation, catalysée au molybdène, d'alcool tertiobutylique et d'oxyde de propylène à partir de propylène et d'hydroperoxyde de tertiobutyle, qui comprend les étapes de :
a. chargement continu d'éthylèneglycol substantiellement anhydre et de dimolybdate d'ammonium substantiellement anhydre dans un réacteur de préparation de catalyseur, et leur mélange dans celui-ci en des quantités suffisantes pour fournir 7 à 20 moles d'éthylèneglycol par atome gramme de molybdène contenu dans ledit dimolybdate d'ammonium,
b. chauffage continu dudit mélange d'éthylèneglycol et de dimolybdate d'ammonium dans ledit réacteur de préparation de catalyseur à une pression de 0,1 à 20,8 MPa (0 à 3000 psig) et une température de réaction de 70 °C à 250 °C pendant 1 à 2 h, suffisant pour former une solution, exempte de solides, d'un complexe d'éthylèneglycol avec ledit dimolybdate d'ammonium,
c. maintien continu de ladite solution, exempte de solides, dudit complexe d'éthylèneglycol avec du dimolybdate d'ammonium dans ledit réacteur de préparation de catalyseur, à une température de 90 °C à 110 °C pendant de 0,5 à 5 h, suffisant pour permettre la vaporisation et l'élimination des sous-produits volatils, y compris l'eau, en une quantité suffisante pour fournir une solution d'éthylèneglycol initiale contenant 16 à 52 % en poids dudit complexe d'éthylèneglycol avec ledit dimolybdate d'ammonium et ayant une teneur en molybdène de 6 à 20 % en poids et une concentration en eau de 0,5 à 6 % en poids.
d. chargement continu d'une solution, dans de l'alcool tertiobutylique, de propylène et d'hydroperoxyde de tertiobutyle dans une zone de réaction d'époxydation avec une quantité catalytique d'un mélange de catalyseurs de (aa) ladite solution initiale d'éthylèneglycol, dudit complexe d'éthylèneglycol avec ledit dimolybdate d'ammonium, avec (bb) une solution finale d'éthylèneglycol, d'un complexe d'éthylèneglycol avec des composés de molybdène contenant de l'ammonium obtenus à partir d'un précipité de composés solides de molybdène contenant de l'ammonium, en des quantités suffisantes pour fournir un rapport molaire initial du propylène à l'hydroperoxyde de tertiobutyle de 1,05:1 à 2:1 et une concentration de 100 à 600 ppm dudit mélange de catalyseurs dans ladite solution d'alcool tertiobutylique,
e. établissement de conditions de réaction d'époxydation dans ladite zone de réaction d'époxydation, incluant une température de 50 °C à 180 °C et une durée de réaction de 1 à 5 h pour faire ainsi réagir ledit propylène avec ledit hydroperoxyde de tertiobutyle et former un produit de réaction d'époxydation comprenant le propylène n'ayant pas réagi, l'hydroperoxyde de tertiobutyle n'ayant pas réagi, de l'oxyde de propylène, de l'alcool tertiobutylique, un complexe de catalyseur molybdène-éthylèneglycol dissous et des impuretés contenant de l'oxygène,
f. Séparation continue dudit produit de réaction d'époxydation dans une zone de distillation en fractions de distillation incluant une fraction de distillat de propylène, une fraction de distillat d'oxyde de propylène, une fraction de distillat d'alcool tertiobutylique et une fraction de distillation liquide lourde composée principalement d'hydroperoxyde de tertiobutyle, d'alcool tertiobutylique, d'impuretés contenant de l'oxygène et de complexe de catalyseur molybdène-éthylèneglycol dissous,
g. chargement continu de ladite fraction de distillation lourde dans une zone de précipitation, et saturation de ladite fraction de distillation lourde dans ladite zone de précipitation avec de l'ammoniac pour former ainsi un produit d'amination liquide contenant un précipité de composés solides de molybdène contenant de l'ammonium,
h. chargement continu dudit produit d'amination dans une zone de séparation et, dans celle-ci, séparation du précipité solide de molybdène contenant de l'ammonium de celui-ci,
i. récupération continue dudit précipité solide de molybdène contenant de l'ammonium,
j. chargement continu dudit précipité solide de molybdène contenant de l'ammonium récupéré dans une zone de régénération de catalyseur et le mélange de celui-ci dans celle-ci avec une quantité d'éthylèneglycol substantiellement anhydre suffisante pour fournir 7 à 20 moles d'éthylèneglycol par atome gramme de molybdène contenu dans ledit précipité solide de molybdène contenant de l'ammonium et former un mélange d'alimentation d'éthylèneglycol,
k. chauffage continu dudit mélange d'alimentation d'éthylèneglycol dans ladite zone de régénération de catalyseur à une pression de 0,1 à 20,8 MPa (0 à 3000 psig) et une température de réaction de 70 °C à 250 °C pendant de 1 à 2 h, suffisant pour former une solution, exempte de solides, d'un complexe d'éthylèneglycol avec les composés contenant de l'ammonium dans le précipité solide de molybdène contenant de l'ammonium,
l. maintien de ladite solution, exempte de solides, dudit complexe d'éthylèneglycol avec lesdits composés contenant de l'ammonium dans ladite zone de régénération de catalyseur à une température de 90 °C à 110 °C pendant de 0,5 à 5 h, suffisant pour permettre la vaporisation et l'élimination des sous-produits volatils, y compris l'eau, en une quantité suffisante pour fournir une solution finale d'éthylèneglycol contenant 16 à 52 % en poids dudit complexe d'éthylèneglycol avec lesdits composés de molybdène contenant de l'ammonium et ayant une teneur en molybdène de 6 à 20 % en poids et une concentration en eau de 0,5 à 6 % en poids, et
m. chargement continu de ladite solution finale d'éthylèneglycol ainsi préparée, d'un complexe d'éthylèneglycol avec des composés de molybdène contenant de l'ammonium, dans ladite zone de réaction d'époxydation, sous la forme de ladite solution finale d'éthylèneglycol, d'un complexe d'éthylèneglycol avec des composés de molybdène contenant de l'ammonium obtenus à partir d'un précipité de composés solides de molybdène contenant de l'ammonium.
